(19) Europäisches Patentamt
European Patent Office
Office européen des brevets

(11) Veröffentlichungsnummer: **0 510 479 A1**

(12) **EUROPÄISCHE PATENTANMELDUNG**

(21) Anmeldenummer: **92106350.9**

(51) Int. Cl.5: **C07D 277/68**, A01N 43/78

(22) Anmeldetag: **13.04.92**

(30) Priorität: **25.04.91 DE 4113421**

(43) Veröffentlichungstag der Anmeldung:
**28.10.92 Patentblatt 92/44**

(84) Benannte Vertragsstaaten:
**BE CH DE FR GB IT LI NL**

(71) Anmelder: **BAYER AG**

**W-5090 Leverkusen 1 Bayerwerk(DE)**

(72) Erfinder: **Wagner, Klaus, Dr.**
**Jakob-Böhme-Strasse 2**
**W-5000 Köln 80(DE)**
Erfinder: **Förster, Heinz, Dr.**
**Am Eckbusch 1**
**W-5600 Wuppertal 1(DE)**
Erfinder: **Schmidt, Robert R., Dr.**
**Im Waldwinkel 110**
**W-5060 Bergisch Gladbach 2(DE)**
Erfinder: **Santel, Hans-Joachim, Dr.**
**Grünstrasse 9a**
**W-5090 Leverkusen(DE)**

(54) **7-Chlor-Benzthiazolyloxyacetamide.**

(57) Die Erfindung betrifft neue 7-Chlor-benzthiazolyloxyacetamide der Formel (I),

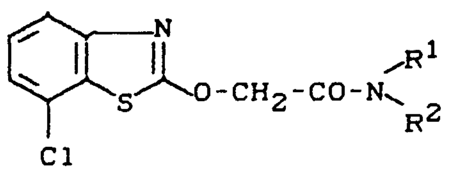

(I)

in welcher

- $R^1$ für Wasserstoff oder für einen gegebenenfalls substituierten Rest aus der Reihe Alkyl, Alkenyl, Alkinyl oder Aralkyl steht,
- $R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder
- $R^1$ und $R^2$ zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann,

ein Verfahren zur ihrer Herstellung und ihre Verwendung als Herbizde.

EP 0 510 479 A1

Die Erfindung betrifft neue 7-Chlor-benzthiazolyloxyacetamide, ein Verfahren zu ihrer Herstellung und ihre Verwendung als Herbizide.

Es ist bereits bekannt, daß bestimmte Benzthiazolyloxyacetamide, wie z.B. Benzthiazolyloxyessigsäure-N-methylanilid und 6-Chlor-benzthiazolyloxyessigsäure-N-methylanilid, herbizide Eigenschaften aufweisen (vgl. EP-A 5501 und EP-A 161602). Die herbizide Wirksamkeit dieser Verbindungen ist jedoch nicht immer ganz zufriedenstellend.

Es wurden nun neue 7-Chlor-benzthiazolyloxyacetamide der allgemeinen Formel (I) gefunden,

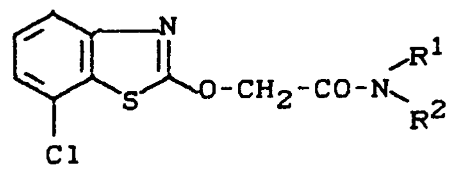

(I)

in welcher

- $R^1$ für Wasserstoff oder für einen gegebenenfalls substituierten Rest aus der Reihe Alkyl, Alkenyl, Alkinyl oder Aralkyl steht,
- $R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder
- $R^1$ und $R^2$ zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann.

Weiter wurde gefunden, daß man die neuen 7-Chlor-benzthiazolyloxyacetamide der Formel (I) erhält, wenn man 2,7-Dichlor-benzthiazol der Formel (II)

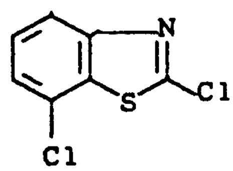

(II)

mit Hydroxyacetamiden der allgemeinen Formel (III)

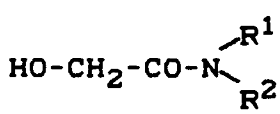

(III)

in welcher

- $R^1$ und $R^2$ die oben angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

Schließlich wurde gefunden, daß die neuen 7-Chlor-benzthiazolyloxyacetamide der Formel (I) interessante herbizide Eigenschaften besitzen.

Überraschenderweise zeigen die erfindungsgemäßen Verbindungen der Formel (I) bei sehr guter Verträglichkeit gegenüber Kulturpflanzen, wie z.B. Reis, wesentlich stärkere Wirkung gegen schwer bekämpfbare Unkräuter, wie z.B. Hühnerhirse, als Benzthiazolyloxyessigsäure-N-methyl-anilid und 6-Chlor-benzthiazolyloxyessigsäure-N-methylanilid, welche chemisch naheliegende bekannte Verbindungen sind.

Gegenstand der Erfindung sind vorzugsweise Verbindungen der Formel (I), in welcher

- $R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,
- $R^2$ für $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$- oder $C_6$-Cycloalkenyl, für Benzyl,

welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder für $C_3$-$C_4$-Alkenyloxy steht, oder

$R^1$ und $R^2$ zusammen mit dem Stickstoffatom, an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist.

Die Erfindung betrifft insbesondere Verbindungen der Formel (I), in welcher

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy oder Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydro(iso)-chinolinyl stehen.

Beispiele für die erfindungsgemäßen Verbindungen der Formel (I) sind in der nachstehenden Tabelle 1 aufgeführt.

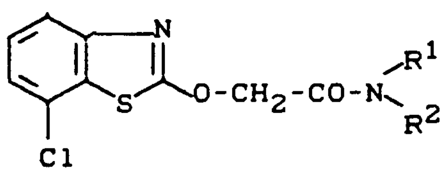

(I)

Tabelle 1: Beispiele für die Verbindungen der Formel (I)

| 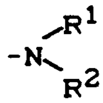 | 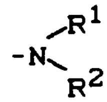 |
|---|---|
| $-N(CH_3)_2$ | 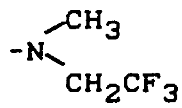 |
| $-N(C_2H_5)_2$ | |
| $-N(C_3H_7)_2$ | 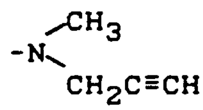 |
| $-N(C_4H_9)_2$ | |
| $-N(CH_2CH{=}CH_2)_2$ | 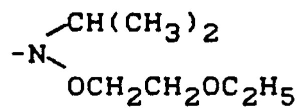 |
| $-N(CH_2C{\equiv}CH)_2$ | |
| 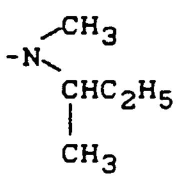 | 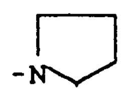 |
| 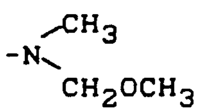 | 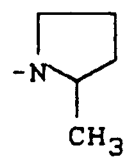 |
| 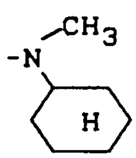 | 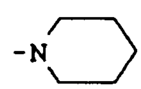 |
| | 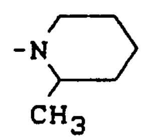 |
| 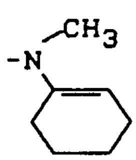 | 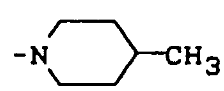 |
| 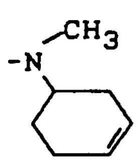 | 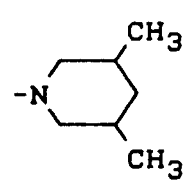 |

Tabelle 1 - Fortsetzung

| 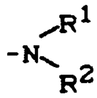 | 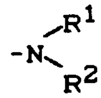 |
| --- | --- |
| 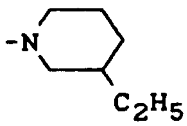 | 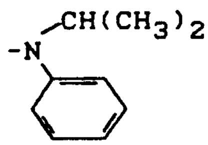 |
| 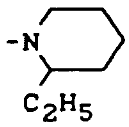 | 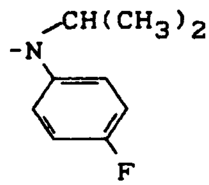 |
| 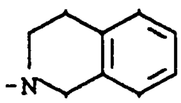 | 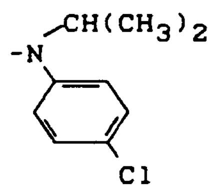 |
| 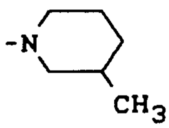 | 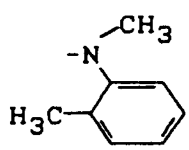 |
| 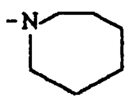 | 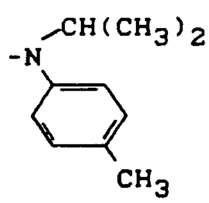 |
| 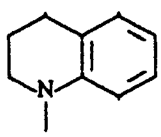 | 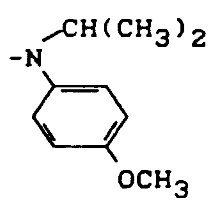 |
| 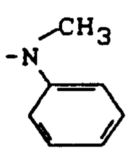 | |
| 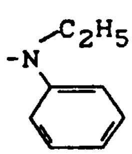 | |

Tabelle 1 - Fortsetzung

| 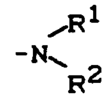 | 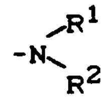 |
|---|---|
| 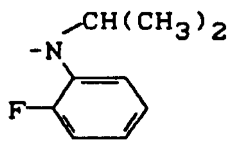 | 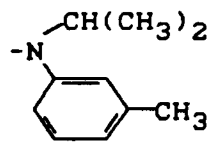 |
| 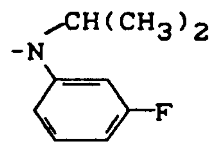 | 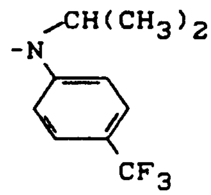 |

Verwendet man beispielsweise 2,7-Dichlor-benzthiazol und Hydroxyessigsäurediethylamid als Ausgangsstoffe, so läßt sich der Reaktionsablauf beim erfindungsgemäßen Herstellungsverfahren durch das folgende Formelschema darstellen:

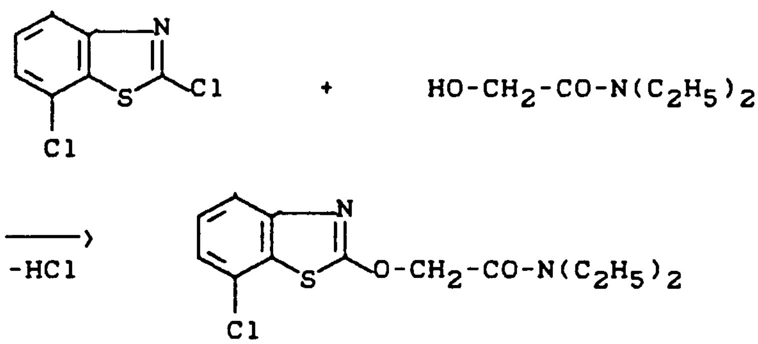

Das beim erfindungsgemäßen Herstellungsverfahren für die Verbindungen der Formel (I) als Ausgangsstoff benötigte 2,7-Dichlor-benzthiazol der Formel (II) ist bereits bekannt (vgl. Canad. J. Chem. 49 (1971), 956-964).

Die beim erfindungsgemäßen Verfahren zur Herstellung von Verbindungen der Formel (I) weiter als Ausgangsstoffe zu verwendenden Hydroxyessigsäureamide sind durch die Formel (III) allgemein definiert.

In Formel (III) haben $R^1$ und $R^2$ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für $R^1$ und $R^2$ angegeben wurden.

Die Hydroxyessigsäureamide der Formel (III) sind bekannt und/oder können nach an sich bekannten Verfahren hergestellt werden (vgl. US-P 4 509 971 und US-P 4 645 525; ferner US-P 4 334 073, DE-OS 3 038 598, DE-OS 3 038 636, EP-A 37 526, EP-A 348 737, DE-OS 38 19 477).

Das erfindungsgemäße Verfahren zur Herstellung der neuen 7-Chlor-benzthiazolyloxyacetamide der Formel (I) wird vorzugsweise unter Verwendung von Verdünnungsmitteln duchgeführt. Hierzu gehören vorzugsweise Kohlenwasserstoffe, wie z. B. Toluol, Xylol oder Cyclohexan, Halogenkohlenwasserstoffe, wie z. B. Methylenchlorid, Ethylenchlorid, Chloroform oder Chlorbenzol, Ether, wie z. B. Diethylether, Dipropylether, Diisopropylether, Dibutylether, Diisobutylether, Glycoldimethylether, Tetrahydrofuran und Dioxan, Alkohole, wie z. B. Methanol, Ethanol, Propanol, Isopropanol oder Butanol, Ketone, wie z. B. Aceton, Methylethylketon, Methylisopropylketon und Methylisobutylketon, Ester, wie z. B. Essigsäuremethylester und Essigsäureethylester, Amide, wie z. B. Dimethylformamid, Dimethylacetamid und N-Methyl-pyrrolidon, Nitrile, wie

z. B. Acetonitril und Propionitril, Sulfoxide, wie z. B. Dimethylsulfoxid sowie Wasser oder wäßrige Salzlösungen.

Als Salze verwendet man hierbei vorzugsweise Chloride oder Sulfate von Alkali- oder Erdalkalimetallen, wie beispielsweise Natriumchlorid, Kaliumchlorid oder Calciumchlorid, Besonders bevorzugt ist Natriumchlorid.

Das erfindungsgemäße Verfahren wird vorteilhaft unter Verwendung von Säurebindemitteln durchgeführt. Als solche werden vorzugsweise stark basische Alkali- und Erdalkalimetallverbindungen, beispielsweise Oxide, wie z. B. Natrium-, Kalium-, Magnesium- und Calciumoxid, Hydroxide, wie z. B. Natrium-, Kalium-, Magnesium- und Calciumhydroxid und/oder Carbonate, wie z. B. Natrium-, Kalium-, Magnesium- und Calciumcarbonat verwendet.

Der Zusatz von 0,01 bis 10 Gew.-% (bezogen auf eingesetztes Glycolsäureamid der Formel (III)) eines Phasentransferkatalysators mag sich in einigen Fällen als vorteilhaft erweisen. Als Beispiele für solche Katalysatoren seien genannt:
Tetrabutylammoniumchlorid, Tetrabutylammoniumbromid, Tributyl-methylphosphoniumbromid, Trimethyl-$C_{13}$/$C_{15}$-alkyl-ammoniumchlorid, Dibenzyl-dimethyl-ammonium-methylsulfat, Dimethyl-$C_{12}$/$C_{14}$-alkyl-benzyl-ammoniumchlorid, Tetrabutylammoniumhydroxid, 18-Krone-6, Triethylbenzylammoniumchlorid, Trimethylbenzylammoniumchlorid, Tetraethylammoniumbromid.

Die Reaktionstemperaturen können bei dem erfindungsgemäßen Verfahren in einem größeren Bereich variiert werden. Im allgemeinen arbeitet man bei Temperaturen zwischen -50 °C und +110 °C, vorzugsweise bei Temperaturen zwischen -20 °C und +80 °C.

Das erfindungsgemäße Verfahren wird im allgemeinen bei Normaldruck durchgeführt, es kann aber auch bei erhöhtem oder vermindertem Druck, etwa zwischen 0,1 und 10 bar, durchgeführt werden.

Zur Durchführung des erfindungsgemäßen Verfahrens setzt man je Mol 2,7-Dichlor-benzthiazol der Formel (II) im allgemeinen 0,5 bis 5 Mol, vorzugsweise 0,8 bis 1,5 Mol, Hydroxyessigsäureamid der Formel (III) ein. Die Reaktionskomponenten können in beliebiger Reihenfolge zusammengegeben werden. Man rührt jeweils das Reaktionsgemisch bis zum Ende der Umsetzung und arbeitet nach üblichen Methoden auf.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind.

Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur Bekämpfung von monokotylen Unkräutern in dikotylen Kulturen, zum Teil auch in monokotylen Kulturen (wie z. B. in Reis). Sie sind vor allem zur Bekämpfung von Schadgräsern (wie z. B. Echinochloa) in Wasserreis geeignet.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen

in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen infrage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, infrage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 20 und 2000 g Wirkstoff pro Hektar Bodenfläche, vorzugsweise zwischen 50 und 1000 g pro ha.

Die Herstellung und die Verwendung der erfindungsgemäßen Wirkstoffe geht aus den nachfolgenden Beispielen hervor.

Herstellungsbeispiele:

Beispiel 1

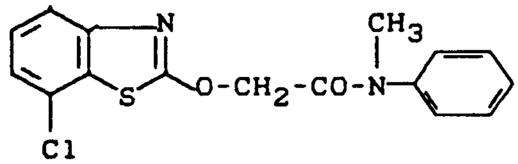

Eine Mischung aus 8,25 g (0,05 Mol) Hydroxyessigsäure-N-methyl-anilid, 3,1 g (0,05 Mol KOH) 90%igem Kaliumhydroxid-Pulver und 100 ml Isopropanol wird auf -20 °C abgekühlt und bei dieser Temperatur wird unter Rühren eine Lösung von 10,2 g (0,05 Mol) 2,7-Dichlor-benzthiazol in 40 ml Acetonitril tropfenweise dazugegeben. Das Reaktionsgemisch wird dann 6 Stunden bei 0 °C bis 10 °C gerührt, anschließend auf 500 ml Wasser gegossen und das kristallin angefallene Produkt durch Absaugen isoliert.

Man erhält 15,9 g (96% der Theorie) (7-Chlor-benzthiazol-2-yl-oxy)-essigsäure-N-methyl-anilid vom Schmelzpunkt 114 °C.

Analog Beispiel 1 und entsprechend der allgemeinen Beschreibung des erfindungsgemäßen Herstellungsverfahrens können beispielsweise auch die in der nachstehenden Tabelle 2 aufgeführten Verbindungen der Formel (I) hergestellt werden.

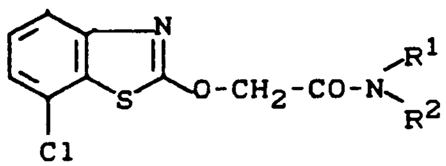

(I)

Tabelle 2: Herstellungsbeispiele für die Verbindungen der Formel (I)

| Bsp.-Nr. | $R^1$ | $R^2$ | Schmelzpunkt (°C) |
|---|---|---|---|
| 2 | $CH(CH_3)_2$ | 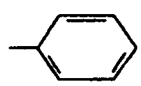 | 106 |
| 3 | $CH(CH_3)_2$ | 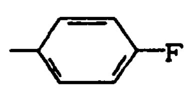 | 105 |
| 4 | $CH_3$ | 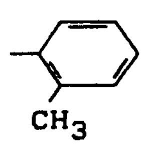 | 126 |
| 5 | $CH_3$ | 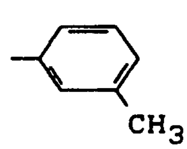 | 140 |

Tabelle 2 (Fortsetzung)

| Bsp.-Nr. | $R^1$ | $R^2$ | Schmelzpunkt (°C) |
|---|---|---|---|
| 6 | $-CH_2-CH=CH_2$ | $-CH_2-CH=CH_2$ | 89 |
| 7 | $-(CH_2)_6-$ | | 102 |
| 8 | $n-C_4H_9$ | $n-C_4H_9$ | (Öl) |
| 9 | $-CH_2-CH_2-\underset{CH_3}{\underset{\vert}{C}}H-CH_2-CH_2-$ | | 96 |
| 10 | $-CH_2-CH=CH_2$ | $-CH=CH-CH_3$ | 57 |
| 11 | $CH_3$ | 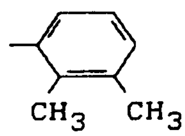 | 138 |
| 12 | $-CH=CH-CH_3$ | $-CH=CH-CH_3$ | (Öl) |

Anwendungsbeispiele:

In den nachfolgenden Anwendungsbeispielen werden die nachstehend aufgeführten Verbindungen als Vergleichssubstanzen herangezogen:

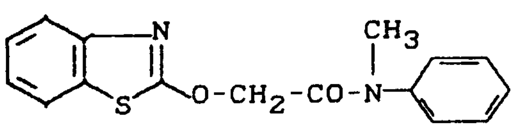

(A)

Benzthiazol-2-yl-oxy-essigsäure-N-methyl-anilid (bekannt aus EP-A 5501, Bsp. 1);

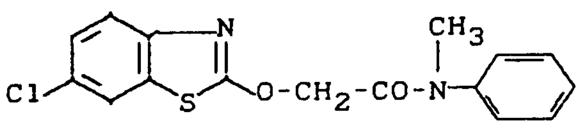

(B)

(6-Chlor-benzthiazol-2-yl-oxy)-essigsäure-N-methylanilid (bekannt aus EP-A 161602, Bsp. 1).

Beispiel A: Pre-emergence-Wasseroberflächenbehandlung bei verpflanztem Wasserreis

Zur Herstellung einer applizierfähigen Zubereitung wird 1 Teil Wirkstoff mit 5 Teilen Aceton gelöst; anschließend wird 1 Teil Benzyloxy-polyglykol-ether als Emulgator zugegeben. Dann wird Wasser bis zur gewünschten Konzentration zugegeben. In Töpfe, die mit Erde gefüllt sind, wird Reis im 2-3-Blattstadium verpflanzt. Samen von Testpflanzen werden ausgelegt (1 cm tief). Zwei Tage später werden die Töpfe mit

Wasser 3 cm überstaut. Anschließend werden die Wirkstoffzubereitungen auf die Wasseroberfläche ausgebracht. 4 Wochen später wird die herbizide Wirkung und die Schädigung der behandelten Pflanzen im Vergleich zu unbehandelten visuell in % ausgewertet. 0% bedeutet keine Wirkung, 100% bedeutet völliges Absterben.

In diesem Test zeigt sich die sehr gute Verträglichkeit der erfindungsgemäßen Wirkstoffe - insbesondere der Verbindung aus Herstellungsbeispiel 1 - in Reis bei im Vergleich mit den bekannten Verbindungen (A) und (B) wesentlich stärkerer Wirkung gegen Unkräuter, insbesondere gegen Hühnerhirse (Echinochloa crus galli).

**Tabelle A**

| Wirkstoff | Aufwandmenge (g/ha) | % Wirkung/Schädigung an verpflanztem Reis | an Echinochloa |
|---|---|---|---|
| 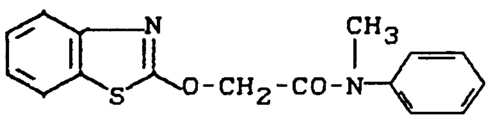 | 500 | 0 | 100 |
| | 250 | 0 | 90 |
| (A) (bekannt) | 125 | 0 | 70 |
| 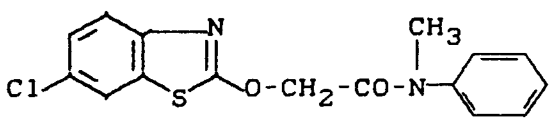 | 500 | 0 | 100 |
| (B) (bekannt) | 250 | 0 | 80 |
| 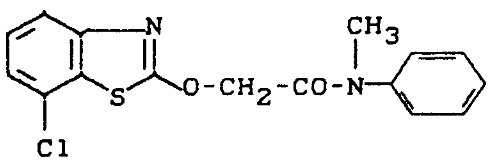 | 500 | 0 | 100 |
| | 250 | 0 | 100 |
| (1) | 125 | 0 | 100 |

## Patentansprüche

1. 7-Chlor-benzthiazolyloxyacetamide der allgemeinen Formel (I),

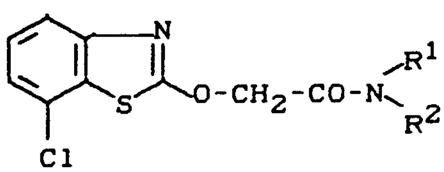

(I)

in welcher

$R^1$ für Wasserstoff oder für einen gegebenenfalls substituierten Rest aus der Reihe Alkyl, Alkenyl, Alkinyl oder Aralkyl steht,

$R^2$ für einen gegebenenfalls substituierten Rest aus der Reihe Alkyl, Alkenyl, Alkinyl, Cycloalkyl, Cycloalkenyl, Aralkyl, Aryl, Alkoxy, Alkenyloxy oder Alkinyloxy steht, oder

$R^1$ und $R^2$ zusammen mit dem Stickstoffatom, an welches sie gebunden sind, einen gegebenenfalls substituierten, gesättigten oder ungesättigten Stickstoff-Heterocyclus bilden, der weitere Heteroatome enthalten kann und an den eine Benzo-Gruppierung anelliert sein kann.

**2.** 7-Chlor-benzthiazolyloxyacetamide der Formel (I) gemäß Anspruch 1, dadurch gekennzeichnet, daß darin

$R^1$ für Wasserstoff, $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl oder für Benzyl steht,

$R^2$ für $C_1$-$C_8$-Alkyl, welches gegebenenfalls durch Fluor, Chlor, Cyano oder $C_1$-$C_4$-Alkoxy substituiert ist, für $C_2$-$C_8$-Alkenyl, welches gegebenenfalls durch Fluor und/oder Chlor substituiert ist, für $C_2$-$C_8$-Alkinyl, für $C_3$-$C_6$-Cycloalkyl, welches gegebenenfalls durch Chlor und/oder $C_1$-$C_3$-Alkyl substituiert ist, für $C_5$- oder $C_6$-Cycloalkenyl, für Benzyl, welches gegebenenfalls durch Fluor, Chlor und/oder $C_1$-$C_4$-Alkyl substituiert ist, für Phenyl, welches gegebenenfalls durch Fluor, Chlor, Brom, Iod, Cyano, Nitro, $C_1$-$C_4$-Alkyl, Trifluormethyl, $C_1$-$C_4$-Alkoxy und/oder $C_1$-$C_4$-Alkylthio substituiert ist, für $C_1$-$C_8$-Alkoxy, welches gegebenenfalls durch $C_1$-$C_4$-Alkoxy substituiert ist, oder für $C_3$-$C_4$-Alkenyloxy steht, oder

$R^1$ und $R^2$ zusammen mit dem Stickstoffatom, an das sie gebunden sind, einen gegebenenfalls ein- bis dreifach durch $C_1$-$C_3$-Alkyl substituierten, gesättigten oder ungesättigten fünf- bis siebengliedrigen Stickstoffheterocyclus bilden, welcher gegebenenfalls benzanelliert ist.

**3.** 7-Chlor-benzthiazolyloxyacetamide der Formel (I) gemäß Anspruch 1, dadurch gekennzeichnet, daß darin

$R^1$ für $C_1$-$C_4$-Alkyl, Allyl oder Propargyl steht,

$R^2$ für $C_1$-$C_6$-Alkyl, $C_1$-$C_2$-Alkoxy-$C_1$-$C_2$-alkyl, Allyl, Propargyl, Cyclopentyl, Cyclohexyl, Cyclohexenyl, Benzyl, Phenyl (welches gegebenenfalls durch Fluor, Chlor, Brom, Cyano, Nitro, Methyl, Ethyl, Trifluormethyl, Methoxy oder Ethoxy substituiert ist), $C_1$-$C_6$-Alkoxy oder $C_1$-$C_2$-Alkoxy-$C_1$-$C_2$-alkoxy steht, oder

$R^1$ und $R^2$ zusammen mit dem Stickstoffatom, an das sie gebunden sind, für gegebenenfalls ein- bis dreifach durch Methyl und/oder Ethyl substituiertes Piperidinyl, für gegebenenfalls ein- oder zweifach durch Methyl und/oder Ethyl substituiertes Pyrrolidinyl, für Perhydroazepinyl oder für 1,2,3,4-Tetrahydro(iso)-chinolinyl stehen.

**4.** (7-Chlor-benzthiazol-2-yl-oxy)-essigsäure-N-methyl-anilid der Formel

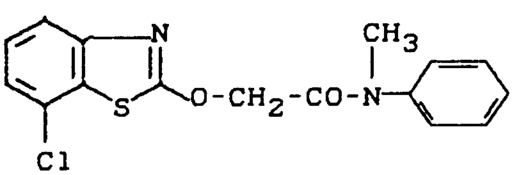

.

**5.** Verfahren zur Herstellung von 7-Chlor-benzthiazolyloxyacetamiden der Formel (I) gemäß Anspruch 1, dadurch gekennzeichnet, daß man

2,7-Dichlor-benzthiazol der Formel (II)

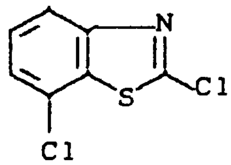

(II)

mit Hydroxyacetamiden der allgemeinen Formel (III)

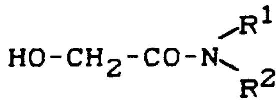

(III)

in welcher

$R^1$ und $R^2$ die in Anspruch 1 angegebenen Bedeutungen haben,

gegebenenfalls in Gegenwart eines Verdünnungsmittels, gegebenenfalls in Gegenwart eines Säurebindemittels und gegebenenfalls in Gegenwart eines Katalysators umsetzt.

**6.** Herbizide Mittel, gekennzeichnet durch einen Gehalt an mindestens einem 7-Chlor-benzthiazolyloxyacetamid der Formel (I) gemäß Anspruch 1.

**7.** Verfahren zur Bekämpfung von Unkräutern, dadurch gekennzeichnet, daß man 7-Chlor-benzthiazolyloxyacetamide der Formel (I) gemäß Anspruch 1 auf die Unkräuter oder ihren Lebensraum einwirken läßt.

**8.** Verwendung von 7-Chlor-benzthiazolyloxyacetamiden der Formel (I) gemäß Anspruch 1 zur Bekämpfung von Unkräutern.

**9.** Verfahren zur Herstellung von herbiziden Mitteln, dadurch gekennzeichnet, daß man 7-Chlor-benzthiazolyloxyacetamide der Formel (I) mit Streckmitteln und/oder oberflächenaktiven Mitteln vermischt.

Europäisches Patentamt

# EUROPÄISCHER RECHERCHENBERICHT

Nummer der Anmeldung

EP 92 10 6350

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,X | EP-A-0 161 602 (BAYER AG)<br>* Ansprüche *<br>--- | 1-9 | C07D277/68<br>A01N43/78 |
| D,X | EP-A-0 005 501 (BAYER AG)<br>* Ansprüche *<br>--- | 1-9 | |
| A | EP-A-0 334 134 (BAYER AG)<br>* Ansprüche *<br>----- | 1-9 | |

| RECHERCHIERTE SACHGEBIETE (Int. Cl.5 ) |
|---|
| C07D |

Der vorliegende Recherchenbericht wurde für alle Patentansprüche erstellt

| Recherchenort | Abschlußdatum der Recherche | Prüfer |
|---|---|---|
| DEN HAAG | 24 JULI 1992 | HENRY J.C. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)